# EUROPÄISCHE PATENTSCHRIFT

(11) **EP 2 215 987 B1**
(45) Veröffentlichungstag und Bekanntmachung des Hinweises auf die Patenterteilung: **27.07.2016**
(21) Anmeldenummer: 10001204.6
(22) Anmeldetag: 05.02.2010
(51) Int. Cl.: A61B 90/30

(54) **Vorrichtung zur Beleuchtung eines Operationsfeldes eines sterilen Operationsraumes**
Device for lighting an operation area of a sterile operating theatre
Dispositif d'éclairage d'un champ opératoire d'une salle d'opération stérile

(30) Priorität: 07.02.2009 DE 102009007986
(43) Veröffentlichungstag der Anmeldung: 11.08.2010
(73) Patentinhaber: Radl, Bernd, Dr., 85283 Wolnzach (DE)
(72) Erfinder: Radl, Bernd, Dr., 85283 Wolnzach (DE)
(74) Vertreter: Liebl, Thomas

(56) Entgegenhaltungen:
- EP-A1- 1 340 470
- EP-A1- 1 568 938
- EP-A1- 1 728 482
- DE-A1- 19 839 827
- FR-A1- 2 889 805
- US-A1- 2005 195 587

## Beschreibung

Die Erfindung betrifft eine Vorrichtung zur Beleuchtung eines Operationsfeldes eines sterilen Operationsraumes nach dem Oberbegriff des Anspruchs 1.

Zur Ausleuchtung von Operationsräumen in zum Beispiel Krankenhäusern ist es allgemein bekannt, neben einem sogenannten Saallicht zusätzlich deckenseitig angeordnete Leuchten vorzusehen, die mittels eines mehrgelenkigen Schwenkarmes schwenkbeweglich im Operationsraum gehaltert sind und von Hand so ausgerichtet werden bzw. im Verlauf einer Operation so neujustiert werden, dass der gewünschte Bereich (Operationsfeld) hinreichend gut beleuchtet bzw. ausgeleuchtet ist. Dabei wird zu Beginn der Operation durch entsprechendes Verschwenken der Leuchten eine gewünschte Ausleuchtung bzw. Beleuchtung des Operationsfeldes eingestellt, in dem die Leuchten an einem mit einem sterilen Kunststoffteil überzogenen Griff erfasst und in die gewünschte Position gezogen werden. Regelmäßig werden dabei zwei Leuchten zu beiden Seiten des Kopfes eines Operateurs relativ kopfnah angeordnet, um die gewünschte gute Aus- bzw. Beleuchtung vornehmen zu können. Wird im Verlauf der Operation eine Neujustierung der Leuchten erforderlich, können diese am sterilen Griff erfasst und in die neue, gewünschte Position gezogen werden. Dies ist relativ zeitaufwendig und mühsam und bedingt gegebenenfalls sogar eine unerwünschte, wenngleich auch in der Regel nur kurze OP-Pause. Zudem besteht aufgrund der relativ kopfnahen Ausrichtung der Leuchten die Gefahr, dass der Operateur mit seinem nicht-sterilen Kopf in Berührung mit dem sterilen Griff der Leuchte bzw. der Leuchten kommt, wodurch diese Griffteile unsteril werden. In diesem Fall muss dann das sterile Kunststoffteil, mit dem der Griff, wie zuvor beschrieben, überzogen ist, ausgetauscht und durch ein steriles Kunststoffteil ersetzt werden. Dies stellt ebenfalls einen unerwünschten zusätzlichen Zeit- und Arbeitsaufwand dar.

Aus der US 2005/0195587 A1 ist eine Vorrichtung bekannt, mit der eine Instrumentenverfolgung realisiert und umgesetzt werden kann. Visuelle Überwachungseinrichtungen sind ferner auch aus der FR 2889805 A1 bekannt.

Die EP 1 728 482 A1 betrifft ein selbsteinstellendes Operationslampensystem, das eine Kamera sowie eine bewegliche Halterung aufweist.

Die EP 1 568 938 A1 betrifft eine Operationsleuchte mit mehreren unterschiedlichen Leuchtmitteln zur Ausleuchtung der Operationsstelle, wobei Mittel zur Farbeinstellung und Farbwiedergabe vorgesehen sind.

Es ist daher Aufgabe der vorliegenden Erfindung, eine Vorrichtung und ein Verfahren zur Beleuchtung eines Operationsfeldes zur Verfügung zu stellen, mittels der bzw. dem die zuvor genannten Nachteile vermieden werden können.

Diese Aufgabe wird gelöst mit den Merkmalen der unabhängigen Ansprüche. Vorteilhafte Ausgestaltungen hierzu sind Gegenstand der darauf rückbezogenen Unteransprüche.

Gemäß Anspruch 1 weist die Vorrichtung zur Beleuchtung eines Operationsfeldes wenigstens eine Leuchte auf, die mittels eines an einer ortsfesten Anlenkstelle eines Wandbereichs, insbesondere eines Deckenwandbereichs, angelenkten Schwenkarmes schwenkbeweglich im Operationsraum gehaltert ist. Weiter weist die Vorrichtung ferner eine mobile Justierhilfe auf, deren jeweilige Position mittels einer Positions-Erfassungseinrichtung dergestalt erfassbar ist, dass die wenigstens eine Leuchte in Richtung auf eine vorgegebene Position, insbesondere auf die Justierhilfe hin ausrichtbar ist. Die Positions-Erfassungseinrichtung ist mittels einer Leuchten-Antriebseinheit der wenigstens einen Leuchte gekoppelt und weist ferner eine Auswerte- und Steuereinrichtung auf oder ist mit einer solchen gekoppelt, mittels der die Leuchten-Antriebseinheit in Abhängigkeit von der jeweils erfassten Position der Justierhilfe angesteuert werden kann. Die Positions-Erfassungseinrichtung ist durch wenigstens eine im Operationsraum installierte Kamera gebildet, mittels der ein definierter Bildbereich erfasst und als Bildsignal zur Positionsermittlung der Auswerte- und Steuereinrichtung zugeführt wird. Hierzu ist die Auswerte- und Steuereinrichtung mittels einer Bilderkennungssoftware ausgestattet, mittels der das von der wenigstens einen Kamera erfasste Bild so auswertbar ist, dass die Auswerte- und Steuereinrichtung die Leuchten-Antriebseinheit zur Leuchtenjustierung in Abhängigkeit von der erfassten Position der Justierhilfe und/oder erfasster Gegenstände und/oder erfasster Personen ansteuert. Erfindungsgemäß ist die Justierhilfe mit einer Zielmarkierung versehen, zum Beispiel mit einer durch einen Kreis oder mehrere konzentrische Kreise ausgebildeten Zielmarkierung, deren Raumausrichtung und/oder Raumposition im von der wenigstens einen Kamera erfassten Bildbereich für eine exakte Leuchtenjustierung dergestalt erfassbar ist, dass eine Abweichung von einem für eine definierte Raumposition vorgegebenen Zielmarkierung-Vergleichsmuster erkannt und die Position und/oder der Einstrahlwinkel der wenigstens einen Leuchte und/oder der Leuchtkegel der wenigstens einen Leuchte entsprechend dem Grad der erfassten Abweichung ausgerichtet wird, insbesondere so bzw. solange ausgerichtet wird, dass die optisch erfasste Zielmarkierung im Wesentlichen mit dem Zielmarkierung-Vergleichsmuster übereinstimmt. Diese Formulierung soll ausdrücklich in einem weiten Sinne verstanden werden, so dass zum Beispiel auch ausdrücklich der Fall umfasst sein soll, bei dem mittels der Justierhilfe ein optisches Signal als Zielmarkierung übertragen wird, zum Beispiel dergestalt, dass die Justierhilfe in einer Grundeinstellung eine erste Farbe anzeigt, die nicht mit einem Farb-Referenzmuster übereinstimmt, so dass eine Farbänderung vom Empfänger erfasst und dementsprechend eine Leuchtenjustierung solange vorgenommen wird, bis die erfasste Farbe mit dem Farb-Referenzmuster übereinstimmt. Die Farbänderung kann dabei grundsätzlich energetisch, zum Beispiel über stromgespeiste Lämpchen, erfolgen. Bevorzugt ist jedoch eine nicht energetische Farbänderung durch zum Beispiel Umlegen einer Klappe oder dergleichen, die auf unterschiedlichen Klappenseiten unterschiedliche Farben aufweist, da damit die Vorrichtung unabhängig von Batterien oder dergleichen zu bedienen ist.

Mit einer derartigen erfindungsgemäßen Lösung kann somit eine einfache vollautomatische Ausrichtung bzw. Neujustierung der jeweiligen Leuchten dadurch erzielt werden, dass diese auf genau die durch die mobile Justierhilfe vorgegebene Position, zum Beispiel ein neu definiertes Operationsfeld oder dergleichen, ausgerichtet wird. Dazu ist die Justierhilfe bevorzugt als im Raum frei bewegliches und/oder in einer definierten Position anordenbares, mobiles Handgerät ausgebildet, wobei besonders bevorzugt eine Einhandbedienung bzw. eine Einhandbetätigung möglich sein soll. Beispielsweise kann ein derartiges Handgerät einfachst vom Operateur in den Bereich bewegt werden bzw. gehalten werden, in dem die Ausleuchtung bzw. Beleuchtung durch die vorgesehenen Leuchten erfolgen soll.

Durch diese erfindungsgemäße Justierhilfe ist es somit nicht mehr erforderlich, dass die wenigstens eine Leuchte von OP-Personal in die jeweils gewünschte Position gebracht wird, was Zeit und Aufwand spart. Des weiteren ist es unerheblich, ob ein Operateur mit dem nicht-sterilen Kopfbereich die Leuchten berührt oder nicht, da die Neujustierung der Lampen von der Leuchten-Antriebseinheit selbsttätig in Abhängigkeit von der erfassten Position der Justierhilfe vorgenommen wird und damit keine Handbedienung der Leuchte erfolgt.

Bevorzugt ist die wenigstens eine Kamera stationär und ortsfest an einer definierten Position im Raum, insbesondere im Bereich oberhalb eines Operationsfeldes eines Operationsraumes, und/oder an der wenigstens einen Leuchte selbst angeordnet.

Gemäß einer weiteren besonders bevorzugten konkreten Ausgestaltung kann die Positions-Erfassungseinrichtung sprachgesteuert über eine Sprachsteuerung oder durch eine mit der Positions-Erfassungseinrichtung signaltechnisch gekoppelte Betätigungseinrichtung, insbesondere einen fußbetätigbaren Fußschalter oder handbetätigbaren Handschalter, aktivierbar sein. Die Betätigungseinrichtung kann weiter beabstandet und entfernt von der Justierhilfe angeordnet sein oder aber auch mitsamt einer Energiequelle integraler Bestandteil der Justierhilfe sein.

Alternativ hierzu kann aber auch hier die Justierhilfe einen Transponder umfassen, bei dessen Aktivierung mittels einer Betätigungseinrichtung ein Justiersignal an die Auswerte- und Steuereinrichtung mit der Maßgabe übermittelt wird, die Leuchtenjustierung anhand der aktuell von der Positions-Erfassungseinrichtung erfassten Position der Justierhilfe vorzunehmen.

An dieser Stelle sei ausdrücklich nochmals erwähnt, dass der Begriff Transponder im Sinne der vorliegenden Erfindung in einem umfassenden Sinne zu verstehen ist, das heißt als Sender, der optische Signale, Funk-, Infrarot- oder Schallwellen bzw. ein Magnetfeld oder dergleichen emittiert und dem ein entsprechender vorrichtungsseitiger Empfänger zugeordnet ist, der mit einer Steuereinrichtung gekoppelt ist. Weiter kann die Übertragung der Signale grundsätzlich auch mittels Kabel erfolgen, wenngleich die kabellose Übertragung bevorzugt ist.

Mit der erfindungsgemäßen Justierhilfe kann sowohl eine Grob- als auch eine Feinjustierung der wenigstens einen Leuchte vorgenommen werden. Die Vorrichtung kann weiter bevorzugt zwischen einem Hand- und einem Automatikbetrieb umgeschalten werden, wobei die wenigstens eine Leuchte bei Aktivierung des Automatikbetriebs und nicht aktivierter Positions-Erfassungseinrichtung bevorzugt in eine Grundstellung bewegt wird.

Weiter kann beispielsweise vorgesehen sein, im Bereich der Vorrichtung, vorzugsweise an der wenigstens einen Leuchte selbst, wenigstens einen Lichtsensor vorzusehen, der das vom Operationsfeld zurückgeworfene Licht erfasst und dadurch feststellen kann, ob sich eine Person und/oder ein Gegenstand für eine definierte Zeitdauer im ausgeleuchteten Bereich befindet. Wird dies bejaht, dann kann von der Auswerte- und Steuereinheit eine Feinjustierung bzw. Nachjustierung dergestalt vorgenommen werden, dass die wenigstens eine Leuchte so positioniert wird, dass das Operationsfeld wieder in der gewünschten Weise ausgeleuchtet wird. Als Führungsgröße für diese Fein- bzw. Nachjustierung kann zum Beispiel die vom Lichtsensor erfasste Lichtintensität in einem definierten Bereich dienen. In analoger Weise kann zusätzlich oder alternativ in Abhängigkeit vom reflektierten Licht entsprechend vorgegebener Parameter eine Änderung der Lichtfarbe bzw. der Lichtzusammensetzung und/oder der Lichtstärke bzw. Helligkeit vorgenommen werden.

Gemäß einer weiteren bevorzugten Ausgestaltung sind wenigstens zwei Leuchten vorgesehen, die mittels der Auswerte- und Steuereinrichtung so angesteuert werden können, dass ein Operationsfeld mittels der jeweiligen Leuchtkegel mit einer vorgegebenen Leuchtfeldgröße ausgeleuchtet werden kann. Das heißt, dass zum Beispiel in Abhängigkeit von dem ermittelten Abstand der Justierhilfe von einem definierten Referenzpunkt, zum Beispiel dem Empfänger bzw. einer Kamera der Fokus der Leuchten eingestellt wird. Besonders bevorzugt weist hierbei die Auswerte- und Steuereinrichtung wenigstens eine Abstandserfassungseinrichtung auf, mittels der der Abstand der Justierhilfe zu der wenigstens einen Kamera als Abstandssignal erfasst wird und dementsprechend eine Leuchtkegelausrichtung und/oder -justierung erfolgt. So kann zum Beispiel während einer Operation durch einfaches Bewegen der Justierhilfe nach zum Beispiel oben oder unten der Fokus der Leuchten verändert und zum Beispiel neu eingestellt werden, das heißt zum Beispiel die Größe des ausgeleuchteten Feldes in der gewünschten Weise variiert werden.

Gemäß einer weiteren besonders bevorzugten Ausgestaltung der vorliegenden Erfindung kann die Vorrichtung mit einer Verstelleinrichtung zur Lageveränderung, insbesondere zur Höhenverstellung, eines Operationstisches gekoppelt sein. Die Kopplung erfolgt dabei bevorzugt dergestalt, dass die Vorrichtung, zum Beispiel mittels einer separaten oder aber auch mit der zuvor beschriebenen Positions-Erfassungseinrichtung, eine Lageveränderung des Operationstisches erfasst und die wenigstens eine Leuchte in Abhängigkeit von vorgegebenen, definierten Lageveränderungs- und/oder Ausleuchtparametern justiert. Je nach dem Grad der Lageveränderung des Operationstisches kann es dabei aber auch bei der Beibehaltung der Leuchtenausrichtung, wie sie voreingestellt ist, bleiben. Eine Neujustierung ist grundsätzlich nur dann erforderlich, wenn das ausgeleuchtete Operationsfeld bezüglich der Beleuchtungsanforderungen nicht mehr ausreichend beleuchtet bzw. ausgeleuchtet sein sollte. Diese Variante wird ausdrücklich auch separat beansprucht, das heißt unabhängig von dem Einsatz einer mobilen Justierhilfe, wenngleich auch die Kombination beider Varianten von Vorteil ist, da dann die Positions-Erfassungseinrichtung, die die Position der mobilen Justierhilfe erfasst, gleichzeitig auch die zum Beispiel Höhenverstellung des Operationstisches erfassen kann.

Weiter kann als zusätzliche Redundanz auch eine Fehlererkennung vorgesehen sein, die bei einer erkannten Störung bzw. bei einem erkannten Ausfall der Vorrichtung einen herkömmlichen "Handbetrieb" freigibt, das heißt die Leuchtenverstellung mittels der Hand ermöglicht.

Die Erfindung wird nachfolgend anhand einer Zeichnung näher erläutert.

Es zeigen:
- Fig. 1: schematisch eine Draufsicht auf einen Operationssaal mit einer erfindungsgemäßen Vorrichtung in einer Beleuchtungs-Ausgangssituation,
- Fig. 2: schematisch eine Draufsicht gemäß Fig. 1 mit gegenüber der Ausgangssituation geänderter neuer Beleuchtungssituation,
- Fig. 3: schematisch die Darstellung gemäß Fig. 2 mit neu ausgerichteten Leuchten,
- Fig. 4: schematisch eine als Handgerät ausgebildete mobile Justierhilfe bei einer Aktivierung des Transponders im Handgerät, und
- Fig. 5: schematisch die zum Beispiel ein Funksignal aussendende aktivierte Justierhilfe.

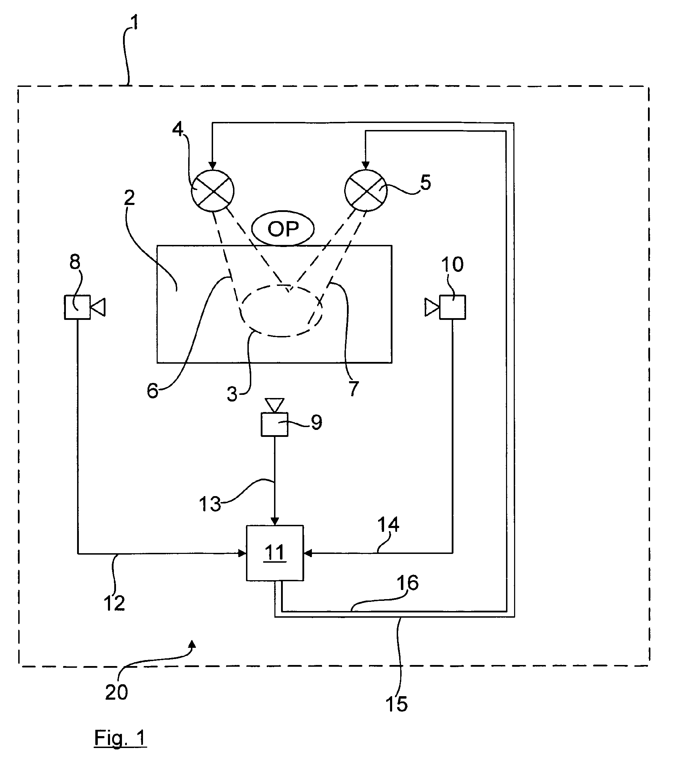

In Fig. 1 ist schematisch eine Draufsicht auf einen Operationsraum 1 mit einem Operationstisch 2 gezeigt, auf dem ein Operationsfeld 3 mittels hier beispielhaft zweier Leuchten 4, 5 ausgeleuchtet wird. Die Leuchten 4, 5 sind in hier nicht dargestellter Weise über Schwenkarme schwenkbeweglich an bevorzugt der Deckenwand gehaltert und angelenkt.

Wie dies aus der Fig. 1 gut ersichtlich ist, sind die beiden Leuchten 4, 5 hier im Wesentlichen zu beiden Seiten eines Operateurs OP angeordnet und strahlen mit ihren Leuchtkegeln 6, 7 von beiden Seiten des Operateurs OP her in Richtung auf das Operationsfeld 3.

Ferner sind im Operationsraum 1 hier beispielhaft drei Kameras 8, 9, 10 ortsfest und stationär, zum Beispiel an Seiten- und/oder Deckenwänden angeordnet, die über eine elektrische Leitungsverbindung mit einer Auswerteund Steuereinrichtung 11 verbunden sind. Die Verbindungsleitungen 12, 13, 14 können zum Beispiel elektrische Leitungen sein. Grundsätzlich kann die Übertragung der Daten von den Kameras 8, 9, 10 zur Auswerte- und Steuereinrichtung 11 aber auch drahtlos erfolgen.

Von der Auswerte- und Steuereinrichtung 11 führen Steuerleitungen 15, 16 hier lediglich schematisch und beispielhaft zu den Leuchten 4, 5 bzw. zu hier nicht dargestellten Antriebseinheiten, die die entsprechende Verstellung der Schwenkarme und der Leuchten bewirken, um diese Leuchten 4, 5 neu zu positionieren bzw. auszurichten. Auch die Signalübertragung über die Steuerleitung 15, 16 kann grundsätzlich ebenfalls kabellos ausgeführt sein.

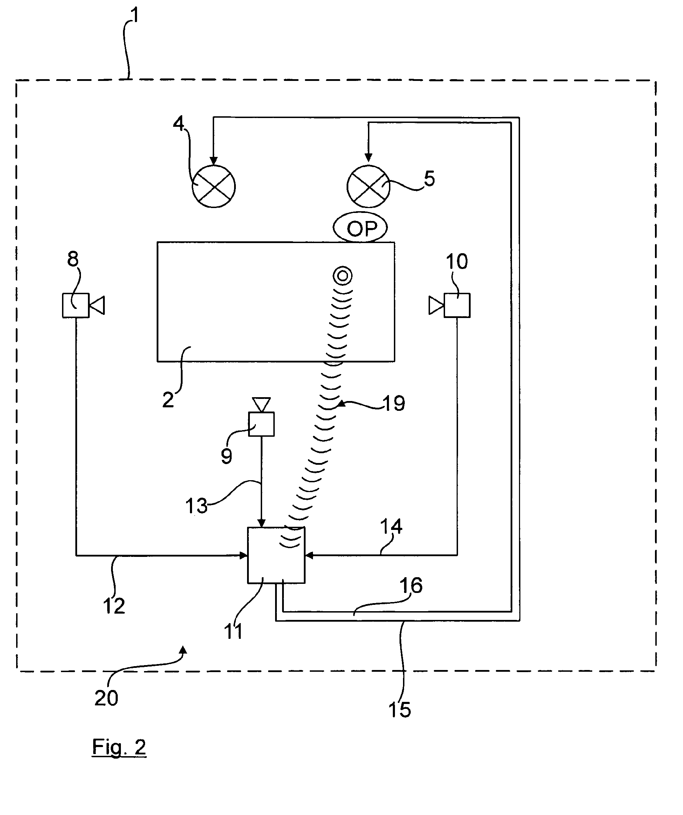

Bewegt sich nunmehr der Operateur OP in eine andere Position, wie in der Fig. 2 gezeigt, ist eine neue Ausrichtung der Leuchten 4, 5 erforderlich, da, wie in der Fig. 2 gezeigt, diese in der Stellung der Fig. 1 verbleiben und somit das hier lediglich aus Übersichtlichkeitsgründen nicht dargestellte Operationsfeld 3 so ausleuchten würden, wie in der Fig. 1 dargestellt.

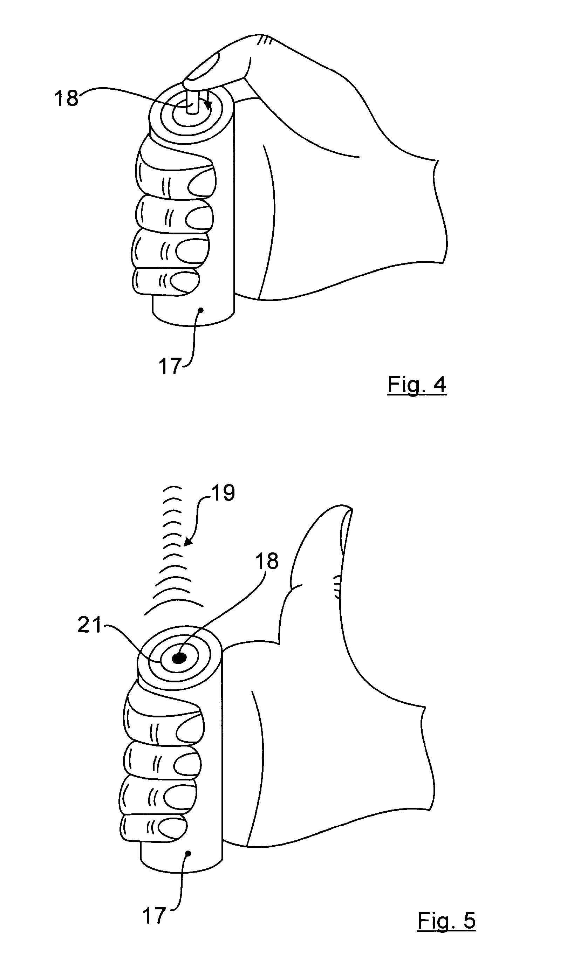

Zur Neujustierung der Leuchten 4, 5 nimmt dann der Operateur OP die in der Fig. 4 schematisch und lediglich beispielhaft dargestellte, ein Handgerät für eine Einhandbedienung ausbildende Justierhilfe 17, in die ein Sender bzw. Transponder integriert sein kann. Wie in der Fig. 2 schematisch dargestellt, wird diese Justierhilfe 17 vom Operateur OP dann über dem Operationstisch 2 in der Position positioniert, in der der Operateur OP die Ausleuchtung bzw. Beleuchtung des Operationsfeldes wünscht. Durch Betätigung eines hier lediglich beispielhaft als Druckknopf 18 dargestellten Betätigungseinrichtung kann dann der Sender bzw. Transponder der Justierhilfe 17 so aktiviert werden, dass dieser ein in der Fig. 5 lediglich schematisch dargestelltes Signal 19, zum Beispiel ein Funksignal, aussendet, das, wie dies insbesondere aus der Fig. 2 ersichtlich ist, von einem Empfänger in der Auswerte- und Steuereinrichtung 11 empfangen wird. Als Reaktion auf dieses Funksignal 19 wird dann die Auswerte- und Steuereinrichtung 11 so aktiviert, dass zum Beispiel eine Bilderkennungssoftware die von den Kameras 8, 9 und 10 ermittelten Bilder auswertet, und zwar dergestalt auswertet, dass damit die genaue Position der Justierhilfe 17 im Operationsraum 1 bestimmt wird. Alternativ hierzu kann die Aktivierung auch über einen Sprachbefehl sprachgesteuert und/oder über einen Fußschalter erfolgen.

Die Justierhilfe weist hier eine zielscheibenartige Kreismarkierung 21 auf, deren Raumausrichtung im von den Kameras 8, 9 und 10 erfassten Bildbereich für eine exakte Leuchtenjustierung dergestalt erfassbar ist, dass eine Abweichung von einem für eine definierte Raumposition vorgegebenen Zielmarkierung-Vergleichsmuster erkannt und die Position der Leuchten 4, 5 und/oder der Leuchtkegel der Leuchten 4, 5 entsprechend dem Grad der erfassten Abweichung ausgerichtet, insbesondere feinjustiert wird.
Die Kameras 8, 9 und 10 sowie die Auswerte- und Steuereinrichtung 11 bilden hier somit eine Positions-Erfassungseinrichtung 20 aus.

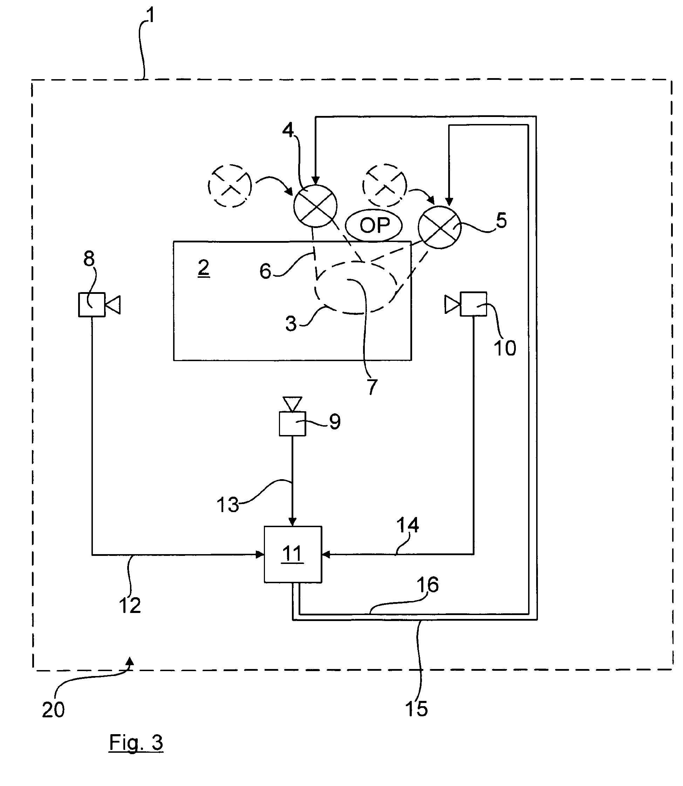

Von der Auswerte- und Steuereinrichtung 11 wird dann in Abhängigkeit von der ermittelten Position der Justierhilfe 17 ein Steuersignal über die Steuerleitung 15, 16 zu den Leuchten-Antriebseinheiten, die hier nicht dargestellt sind, übermittelt, die dann die Leuchten 4, 5, wie in der Fig. 3 dargestellt, in eine solche neue Position bewegt, dass damit das vom Operateur OP bestimmte neue Operationsfeld 3' in der gewünschten Weise ausgeleuchtet wird. Die Ausrichtung der Leuchten 4, 5 bzw. der Leuchtkegel 6, 7 der Leuchten 4, 5 erfolgt hierbei in Abhängigkeit von vorgegebenen Parametern, zum Beispiel kennliniengesteuert.

Alternativ zu der hier dargestellten Ausgestaltung der Positions-Erfassungseinrichtung 20 mit Kameras 8, 9, 10 kann auch vorgesehen sein, anstelle der Kameras Sende- und Empfangsstationen vorzusehen, die das von der Justierhilfe 17 reflektierte Signal zur Positionserkennung der Justierhilfe 17 auswerten. sind, übermittelt, die dann die Leuchten 4, 5, wie in der Fig. 3 dargestellt, in eine solche neue Position bewegt, dass damit das vom Operateur OP bestimmte neue Operationsfeld 3' in der gewünschten Weise ausgeleuchtet wird. Die Ausrichtung der Leuchten 4, 5 bzw. der Leuchtkegel 6, 7 der Leuchten 4, 5 erfolgt hierbei in Abhängigkeit von vorgegebenen Parametern, zum Beispiel kennliniengesteuert.

Alternativ zu der hier dargestellten Ausgestaltung der Positions-Erfassungseinrichtung 20 mit Kameras 8, 9, 10 kann auch vorgesehen sein, anstelle der Kameras Sende- und Empfangsstationen vorzusehen, die das von der Justierhilfe 17 reflektierte Signal zur Positionserkennung der Justierhilfe 17 auswerten.

## Patentansprüche

1. Vorrichtung zur Beleuchtung eines Operationsfeldes eines Operationsraumes, mit wenigstens einer Leuchte, die mittels eines an einer ortsfesten Anlenkstelle eines Wandbereichs angelenkten Schwenkarmes schwenkbeweglich im Raumgehaltert ist,
wobei die Vorrichtung ferner eine mobile Justierhilfe (17) aufweist, deren jeweilige Position mittels einer Positions-Erfassungseinrichtung (20) dergestalt erfassbar ist, dass die wenigstens eine Leuchte (4, 5) in Richtung auf die Justierhilfe (17) hin ausrichtbar ist,
wobei die Positions-Erfassungseinrichtung (20) mittels einer Leuchten-Antriebseinheit der wenigstens einen Leuchte (4, 5) gekoppelt ist und ferner eine Auswerte- und Steuereinrichtung (11) aufweist oder mit einer solchen gekoppelt ist, mittels der die Leuchten-Antriebseinheit in Abhängigkeit von der jeweils erfassten Position der Justierhilfe (17) ansteuerbar ist,
wobei die Positions-Erfassungseinrichtung (20) wenigstens eine im Operationsraum (1) installierte Kamera (8, 9, 10) aufweist, mittels der ein definierter Bilderfassungsbereich erfassbar und als Bildsignal zur Positionsermittlung der Auswerte- und Steuereinrichtung (11) zuführbar ist, und
wobei die Auswerte- und Steuereinrichtung (11) mittels einer Bilderkennungssoftware ausgestattet ist, mittels der das von der wenigstens einen Kamera (8, 9, 10) erfasste Bild so auswertbar ist, dass die Auswerte- und Steuereinrichtung (11) die Leuchten-Antriebseinheit zur Leuchtenjustierung in Abhängigkeit von der erfassten Position der Justierhilfe (17) und/oder erfasster Gegenstände und/oder erfasster Personen ansteuert,
**dadurch gekennzeichnet,**
**dass** die Justierhilfe (17) mit einer Zielmarkierung versehen ist, deren Raumausrichtung und/oder Raumposition im von der wenigstens einen Kamera (8, 9, 10) erfassten Bildbereich für eine Leuchtenjustierung dergestalt erfassbar ist, dass eine Abweichung von einem für eine definierte Raumposition vorgegebenen Zielmarkierung-Vergleichsmuster erkannt und die Position und/oder der Einstrahlwinkel der wenigstens einen Leuchte (4, 5) und/oder der Leuchtkegel der wenigstens einen Leuchte (4, 5) entsprechend dem Grad der erfassten Abweichung so oder solange ausgerichtet wird, dass die optisch erfasste Zielmarkierung im Wesentlichen mit dem Zielmarkierung-Vergleichsmuster übereinstimmt.

2. Vorrichtung nach Anspruch 1, **dadurch gekennzeichnet, dass** die Justierhilfe (17) als im Raum frei bewegliches und/oder in einer definierten Position anordenbares bzw. mobiles Handgerät, insbesondere für eine Einhandbedienung und/oder Einhandbetätigung, ausgebildet ist.

3. Vorrichtung nach einem der vorhergehenden Ansprüche, **dadurch gekennzeichnet, dass** die wenigstens eine Kamera (8, 9, 10) stationär und ortsfest an einer definierten Position im Raum (1), insbesondere im Bereich oberhalb eines Operationsfeldes (3, 3') eines Operationsraumes (1), und/oder an der wenigstens einen Leuchte (4, 5) angeordnet ist.

4. Vorrichtung nach einem der vorhergehenden Ansprüche, **dadurch gekennzeichnet, dass** die Positions-Erfassungseinrichtung (20) sprachgesteuert über eine Sprachsteuerung oder durch eine mit der Positions-Erfassungseinrichtung (20) signaltechnisch gekoppelte Betätigungseinrichtung (18), insbesondere einen Fußschalter oder Handschalter, aktivierbar ist.

5. Vorrichtung nach einem der vorhergehenden Ansprüche, **dadurch gekennzeichnet, dass** die Betätigungseinrichtung beabstandet und entfernt von der Justierhilfe (17) angeordnet ist oder vorzugsweise mitsamt einer Energiequelle integraler Bestandteil der Justierhilfe (17) ist.

6. Vorrichtung nach einem der vorhergehenden Ansprüche, **dadurch gekennzeichnet, dass** im Bereich der Vorrichtung, vorzugsweise an der wenigstens einen Leuchte (4, 5) selbst, wenigstens ein Lichtsensor angeordnet ist, der das vom Operationsfeld zurückgeworfene Licht erfasst, wobei in Abhängigkeit vom erfassten Ergebnis eine Neuund/oder Fein- und/oder Nachjustierung der wenigstens einen Leuchte und/oder eine Änderung der Lichtfarbe bzw. der Lichtzusammensetzung und/oder eine Änderung der Helligkeit der wenigstens einen Leuchte entsprechend vorgegebener Parameter vorgenommen wird, bei der als Führungsgröße für die Neu- und/oder Fein- und/oder Nachjustierung und/oder die Änderung der Lichtfarbe bzw. der Lichtzusammensetzung und/oder der Helligkeit bevorzugt die vom Lichtsensor erfasste Lichtintensität dient.

7. Vorrichtung nach einem der vorhergehenden Ansprüche, **dadurch gekennzeichnet, dass** die Auswerte- und Steuereinrichtung (11) wenigstens eine Abstandserfassungseinrichtung aufweist, mittels der der Abstand der Justierhilfe (17) zu der wenigstens einen Kamera (8, 9, 10) als Abstandssignal erfassbar ist und dementsprechend eine Leuchtkegelausrichtung und/oder -justierung der wenigstens einen Leuchte (4, 5) erfolgt.

8. Vorrichtung nach einem der vorhergehenden Ansprüche, **dadurch gekennzeichnet, dass** die Vorrichtung mit einer Verstelleinrichtung zur Lageveränderung, insbesondere zur Höhenverstellung, eines Operationstisches gekoppelt ist dergestalt, dass die Vorrichtung eine Lageveränderung des Operationstisches, insbesondere mittels der oder einer Positions-Erfassungseinrichtung, erfasst und die wenigstens eine Leuchte (4, 5) in Abhängigkeit von definierten, vorgegebenen Lageveränderungs- und/oder Ausleuchtungsparametern justiert.

9. Vorrichtung nach einem der vorhergehenden Ansprüche, **dadurch gekennzeichnet, dass** die Vorrichtung eine Einrichtung zur Fehlererkennung aufweist, mittels der eine Abweichung von einer Referenzposition der wenigstens einen Leuchte (4, 5) erfassbar ist, vorzugsweise dergestalt erfassbar ist, dass bei einem einen definierten Fehlervergleichswert erreichenden oder übersteigenden Fehler eine Umschaltung von einem Automatikbetrieb auf einen Handbetrieb erfolgt.

## Claims

1. Device for lighting an operation area of an operating theatre, having at least one lamp which, by means of a swivelling arm attached at a fixed attachment point of a wall area, is held such that it can be moved and swivelled in the room,
where the device further has a mobile adjustment aid (17), the respective position of which can be detected by means of a position detection device (20) in such a way that the at least one lamp (4, 5) can be aligned in the direction of the adjustment aid (17),
where the position detection device (20) is coupled to the at least one lamp (4, 5) by means of a lamp drive unit and also has an evaluation and control device (11) or is coupled to one such, by means of which the lamp drive unit can be driven on the basis of the respectively detected position of the adjustment aid (17),
where the position detection device (20) has at least one camera (8, 9, 10) installed in the operating theatre (1), by means of which a defined image detection area can be detected and can be fed to the evaluation and control device (11) as an image signal for the position determination, and
where the evaluation and control device (11) is equipped by means of image recognition software, by means of which the image detected by the at least one camera (8, 9, 10) can be evaluated in such a way that the evaluation and control device (11) drives the lamp drive unit for lamp adjustment as a function of the detected position of the adjustment aid (17) and/or detected objects and/or detected individuals, **characterized in that**
the adjustment aid (17) is provided with a target mark, of which the spatial alignment and/or spatial position in the image area detected by the at least one camera (8, 9, 10) can be detected for lamp adjustment in such a way that a deviation from a target mark comparative pattern predefined for a defined spatial position is detected, and the position and/or the irradiation angle of the at least one lamp (4, 5) and/or the cone of light from the at least one lamp (4, 5) is aligned in accordance with the level of the detected deviation so that or until the optically detected target mark substantially coincides with the target mark comparative pattern.

2. Device according to Claim 1, **characterized in that** the adjustment aid (17) is formed as a handheld instrument that is freely moveable in the theatre and/or can be arranged in a defined position or is mobile, in particular for one-hand operation and/or one-hand actuation.

3. Device according to one of the preceding claims, **characterized in that** the at least one camera (8, 9, 10) is arranged to be stationary and fixed at a defined position in the theatre (1), in particular in the area above an operation area (3, 3') of an operating theatre (1), and/or is arranged on the at least one lamp (4, 5).

4. Device according to one of the preceding claims, **characterized in that** the position detection device (20) can be activated under speech control via a speech control system or by means of an actuating device (18) with a signal connection to the position detection device (20), in particular a foot switch or hand switch.

5. Device according to one of the preceding claims, **characterized in that** the actuating device is arranged at a distance and remote from the adjustment aid (17) or, preferably, together with a power source, is an integral constituent part of the adjustment aid (17).

6. Device according to one of the preceding claims, **characterized in that,** in the area of the device, preferably on the at least one lamp (4, 5) itself, there is arranged at least one light sensor which detects the light reflected from the operation area, wherein, depending on the detected result, new and/or precise and/or re-adjustment of the at least one lamp and/or a change in the light colour or the light composition and/or a change in the brightness of the at least one lamp is performed in accordance with a predefined parameter, in which the reference variable used for the new and/or precise and/or re-adjustment and/or the change in the light colour or the light composition and/or the brightness is preferably the light intensity detected by the light sensor.

7. Device according to one of the preceding claims, **characterized in that** the evaluation and control device (11) has at least one distance detection device, by means of which the distance of the adjustment aid (17) to the at least one camera (8, 9, 10) can be detected as a distance signal and, accordingly, an alignment and/or adjustment of the cone of light from the at least one lamp (4, 5) is carried out.

8. Device according to one of the preceding claims, **characterized in that** the device is coupled to a displacement device for changing the position, in particular for the vertical displacement, of an operating table, in such a way that the device detects a change in the position of the operating table, in particular by means of the or a position detection device, and the at least one lamp (4, 5) is adjusted on the basis of defined, stipulated position-change and/or lighting parameters.

9. Device according to one of the preceding claims, **characterized in that** the device has a means for fault detection, by means of which a deviation from a reference position of the at least one lamp (4, 5) can be detected, preferably can be detected in such a way that, in the event of a fault that reaches or exceeds a defined fault comparison value, a change from automatic operation to manual operation is carried out.

## Revendications

1. Dispositif d'éclairage d'un champ opératoire d'une salle d'opération avec au moins une lampe, qui est maintenue mobile en pivotement dans l'espace au moyen d'un bras pivotant articulé sur un point d'articulation fixe d'une zone murale,
le dispositif comprenant en outre une aide de réglage mobile (17), dont la position respective peut être saisie au moyen d'un système de saisie de position (20) de telle manière qu'au moins une lampe (4,5) peut être orientée en direction de l'aide de réglage (17),
le système de saisie de position (20) étant couplé au moyen d'une unité de commande de lampes de l'au moins une lampe (4,5) et comportant en outre un système d'évaluation et de commande (11) ou étant couplé avec un appareil analogue, au moyen duquel l'unité de commande de lampes peut être activée en fonction de la position respectivement saisie de l'aide de réglage (17),
le système de saisie de position (20) comportant au moins une caméra (8, 9, 10) installée dans la salle d'opération (1), au moyen de laquelle une zone de saisie d'images définie peut être saisie et peut être délivrée au système d'évaluation et de commande (11), en tant que signal vidéo pour la détermination de la position, et
le système d'évaluation et de commande (11) étant doté d'un logiciel d'identification d'images, au moyen duquel l'image saisie par l'au moins une caméra (8, 9, 10) peut être évaluée de telle manière que le système d'évaluation et de commande (11) active l'unité de commande de lampes pour le réglage des lampes en fonction de la position saisie de l'aide de réglage (17) et/ou des objets saisis et/ou des personnes saisies,
**caractérisé en ce que**
l'aide de réglage (17) est dotée d'un repérage de visée dont l'orientation spatiale et/ou la position spatiale dans la zone d'image saisie par l'au moins une caméra (8, 9, 10) pour un réglage de lampe peut être saisie de telle manière qu'un écart par rapport à un modèle de comparaison de repérage de visée prédéfini pour une position spatiale définie est identifié et la position et/ou l'angle de faisceau de l'au moins une lampe (4, 5) et/ou le cône de lumière de l'au moins une lampe (4, 5) selon le degré de l'écart saisi sont orientés de telle manière ou tant que le repérage de visée optiquement saisi coïncide pour l'essentiel avec le modèle de comparaison du repérage de visée.

2. Dispositif selon la revendication 1, **caractérisé en ce que** l'aide de réglage (17) est constituée comme appareil manuel librement mobile dans l'espace et/ou pouvant être disposé ou mobile dans une position définie, en particulier pour s'en servir d'une seule main et/ou l'actionner d'une seule main.

3. Dispositif selon l'une quelconque des revendications précédentes, **caractérisé en ce que** l'au moins une caméra (8, 9, 10) est disposée de manière stationnaire et fixe dans une position définie dans l'espace (1), en particulier dans une zone au-dessus d'un champ opératoire (3, 3') d'une salle d'opération (1) et/ou sur au moins une lampe (4, 5).

4. Dispositif selon l'une quelconque des revendications précédentes, **caractérisé en ce que** le système de saisie de position (20) peut être activé à la voix par une commande vocale ou par un dispositif de commande (18) couplé techniquement par signaux au système de saisie de position (20), en particulier un interrupteur à pédale ou un interrupteur manuel.

5. Dispositif selon l'une quelconque des revendications précédentes, **caractérisé en ce que** le dispositif d'actionnement est disposé à distance et éloigné de l'aide de réglage (17) ou fait de préférence partie intégrante de l'aide de réglage (17) avec une source d'énergie.

6. Dispositif selon l'une quelconque des revendications précédentes, **caractérisé en ce que** dans la zone du dispositif, de préférence sur au moins une lampe (4, 5) proprement dite, est disposé au moins un capteur de lumière qui saisit la lumière renvoyée par le champ opératoire, un réglage nouveau et/ou précis et/ou reréglage de l'au moins une lampe et/ou une modification de la couleur de lumière ou de la composition de la lumière et/ou une modification de la luminosité de l'au moins une lampe selon les paramètres prédéfinis étant effectués en fonction du résultat saisi, pour lesquels réglages et modifications de préférence l'intensité lumineuse saisie par le capteur de lumière sert de grandeur de référence pour le réglage nouveau et/ou précis et/ou reréglage et/ou la modification de la couleur de la lumière ou de la composition de la lumière et/ou de la luminosité.

7. Dispositif selon l'une quelconque des revendications précédentes, **caractérisé en ce que** le système d'évaluation et de commande (11), comporte au moins un dispositif de saisie de distance au moyen duquel la distance de l'aide de réglage (17) par rapport à l'au moins une caméra (8, 9, 10) peut être saisie en tant que signal de distance et une orientation et/ou un réglage du cône de lumière de l'au moins une lampe (4, 5) a lieu en conséquence.

8. Dispositif selon l'une quelconque des revendications précédentes, **caractérisé en ce que** le dispositif est couplé avec un dispositif de réglage pour modifier la position, en particulier pour le réglage de la hauteur d'une table d'opération, de telle manière que le dispositif saisit une modification de position de la table d'opération, en particulier au moyen du ou d'un système de saisie de position et ajuste l'au moins une lampe (4, 5) en fonction de paramètres de modification de position et/ou de couverture lumineuse définis, préalablement établis.

9. Dispositif selon l'une quelconque des revendications précédentes, **caractérisé en ce que** le dispositif comporte un système pour identifier les erreurs au moyen duquel un écart d'une position de référence de l'au moins une lampe (4, 5) peut être saisi, de préférence saisi de telle manière que pour une erreur atteignant ou dépassant une valeur de comparaison d'erreur définie un basculement de fonctionnement automatique en fonctionnement manuel a lieu.
